# EUROPEAN PATENT APPLICATION

(11) **EP 4 230 031 A1**
(43) Date of publication of application: **23.08.2023**
(21) Application number: 22170959.5
(22) Date of filing: 29.04.2022
(51) Int. Cl.: A01K 1/00, A61L 9/22, A01K 1/01

(54) **A PLASMA DEODORANT PET TOILET**

(30) Priority: 21.02.2022 CN 202210157784
(71) Applicant: Shenghui Electronic Technology (Guangdong) Co., Ltd., Dongguan City, Guangdong Province 523000 (CN)
(72) Inventor: Zeng, Weiqiang, Yongfu County, 541800 (CN); Zheng, Weihao, Lufeng City, 516500 (CN)
(74) Representative: Sach, Greg Robert

(57) **Abstract**

The present disclosure relates to the technical field of pet supplies, in particular to a plasma deodorant pet toilet, which comprises a base (1), a cat litter tray (2), an upper cover (3) and a deodorant and sterilization component, wherein the upper cover (3) is installed above the base (1) and forms a basin space (4) with the base (1), the cat litter tray (2) is carried on the base (1), the base (1) is arranged with an open end, the cat litter tray (2) passes through the open end, and the deodorant and sterilization component comprises a plasma generator (6) and an exhaust device (7), the plasma generator (6) and the exhaust device (7) are both arranged on the upper cover, the plasma generator (6) is used to deodorize and sterilize the basin space (4), the exhaust device (7) is used to discharge the gas in the basin space (4). The disclosure can play the roles of eliminating smoke, removing dust, eliminating odor, improving air quality, promoting blood circulation, and preventing bacterial regeneration, which is beneficial to protect the health of humans and cats, has good ventilation, effectively prevents bacteria from growing and reduces odor. It is easy to replace the cat litter, improve the convenience, and have a good user experience.

## Description

### TECHNICAL FIELD

The present disclosure relates to the technical filed of pet supplies, and in particular to a plasma deodorant pet toilet.

### BACKGROUND

The cat litter box is a device used to store cat litter. At the same time, it is used in conjunction with pet cats to use cat litter. It is widely used in household occasions where pet cats are raised, and has a good use effect. People's demand for cat litter boxes has gradually increased, but the existing cat litter boxes cannot achieve effective sterilization and disinfection functions, and are prone to bacterial growth and odor. The cat litter particles float in the air for a long time, which is harmful to the cleaner. It has adverse effects on the respiratory system and is not conducive to the physical and mental health of humans and cats.

### SUMMARY

The technical problem to be solved by the present disclosure is to provide a plasma deodorant pet toilet, which can eliminate smoke, remove dust, eliminate odor, improve air quality, promote blood circulation, prevent bacterial regeneration, and is beneficial to protect the health of humans and cats, good ventilation, effectively prevent bacterial growth and reduce odor, easy to replace cat litter, improve convenience, and good use experience.

In order to solve the technical problem mentioned above, the present disclosure adopts the following technical solutions.

A plasma deodorant pet toilet, comprises a base, a cat litter tray, an upper cover and a deodorant and sterilization component, wherein the upper cover is installed above the base and forms a basin space with the base, the cat litter tray is carried on the base, the base is arranged with an open end, the cat litter tray passes through the open end, and the deodorant and sterilization component comprises a plasma generator and an exhaust device, the plasma generator and the exhaust device are both arranged on the upper cover, the plasma generator is used to deodorize and sterilize the basin space, and the exhaust device is used to blow the positive and negative ions generated by the plasma generator to the basin space; an outside of the cat litter tray is arranged with a handle groove, and the handle groove is a hidden groove structure with an opening downward; the cat litter tray is arranged with a stop flange, the upper cover is arranged with a stop sidewall, and the stop flange is abutted against the stop sidewall.

Further, a side wall of the base is double wall structure, the double wall structure is arranged with inner groove, the inner groove is arranged with a plurality of convex buckles, a lower end of the upper cover is arranged with a plurality of pins, the pins are inserted into the inner groove, and the convex buckles are in one-to-one correspondence with the pins, the pins are arranged with button holes, and the button holes are fastened and installed with the convex buckles.

Further, an upper edge of the cat litter tray is bent and arranged with folded edge, and an inner wall of the upper cover is arranged with limit rib, the folded edge is located under the limit rib, and the limit rib is used to limit the position of the cat litter tray.

Further, the cat litter tray is arranged with a filter sand part, the cat litter tray is arranged with two installation slots, one installation slot is arranged on one inner side wall of the cat litter tray, and the other installation slot is arranged on the other inner side wall of the cat litter tray, the filter sand part is arranged with a filter plate and a card plate, the card plate is bent and arranged on the filter plate, the filter plate is arranged with a plurality of filter holes, one end of the card plate protrudes into one installation slot, and the other end of the card plate protrudes into the other installation slot.

Further, both side walls of the upper cover are concavely arranged with a cavity, and the cavity is arranged with a hook, and the hook is used for hanging a cat litter shovel.

Further, the upper cover is rotatably installed with a door, the upper cover is arranged with an inlet and outlet, and the door is arranged at the inlet and outlet.

Further, the upper cover is arranged with a hidden air inlet, the upper cover is arranged with a mounting plate, and the mounting plate is arranged with an air outlet, the exhaust device is arranged on the mounting plate, an intake end of the exhaust device is arranged corresponding to the hidden air inlet, and an outlet end of the exhaust device is arranged corresponding to the hidden air inlet , the air outlet is communicated with the basin space, the hidden air inlet is formed by a circular array of a plurality of arched convex strips, and there is a ventilation vertical hole between the arched convex strips and a top wall of the upper cover, a plane where the vertical ventilation holes are located is arranged intersecting with the top plane of the upper cover.

Further, a bottom wall of the base is arranged with a plurality of through holes.

Beneficial effects of the present disclosure:
1. The plasma generator can eliminate smoke, remove dust, eliminate odor, improve air quality, promote blood circulation, and prevent bacterial regeneration in the basin space, which is beneficial to protect the health of humans and cats;
2. Opening the exhaust device can make the basin space circulate air, the ventilation effect is good, effectively prevent the growth of bacteria and reduce odor, and the deodorization effect is good;
3. The cat litter tray is a pull-out tray structure, which is easy to replace cat litter, improves convenience, and has a good user experience.

### BRIEF DESCRIPTION OF THE DRAWINGS

FIG. 1 is a schematic diagram of the overall three-dimensional structure of the present disclosure.
FIG. 2 is a schematic structural diagram of the hidden air inlet and the upper cover of the present disclosure.
FIG. 3 is a schematic diagram of a cross-sectional structure of the present disclosure from a first perspective.
FIG. 4 is a schematic diagram of a cross-sectional structure of the present disclosure from a second perspective.
FIG. 5 is a partial enlarged structural schematic diagram of part A in FIG. 4.
FIG. 6 is a schematic diagram of a cross-sectional structure of the present disclosure from a third perspective.
FIG. 7 is a schematic structural diagram of the sand filter component and the cat litter tray of the present disclosure.
FIG. 8 is a schematic diagram of the base and the partially exploded structure of the base of the present disclosure.
FIG. 9 is a schematic diagram of a display, a central controller, a heater, an exhaust device and a plasma generator of the present disclosure.

### Reference Number:

1-base; 2-cat litter tray; 3-upper cover; 4-basin space; 5-installation plate; 6-plasma generator; 7-exhaust device; 8-through hole; 9-double wall structure; 10-inner groove; 11-convex buckle; 12-pin; 13-button hole; 14-hemming; 15-limit rib; 16-filter sand part; 17-installation slot; 18-filter plate; 19-card plate; 20-filter hole; 21-handle groove; 22-stop flange; 23-stop sidewall; 24-cavity; 25-hook; 26-door; 27-hidden air inlet; 28-arched convex strip; 29-ventilation vertical hole; 30-key; 31-display; 32-air outlet; 34-open end.

### DETAILED DESCRIPTION

In order to facilitate the understanding of those skilled in the art, the present disclosure will be further described below with reference to the embodiments and the accompanying drawings, and the contents mentioned in the embodiments are not intended to limit the present disclosure.

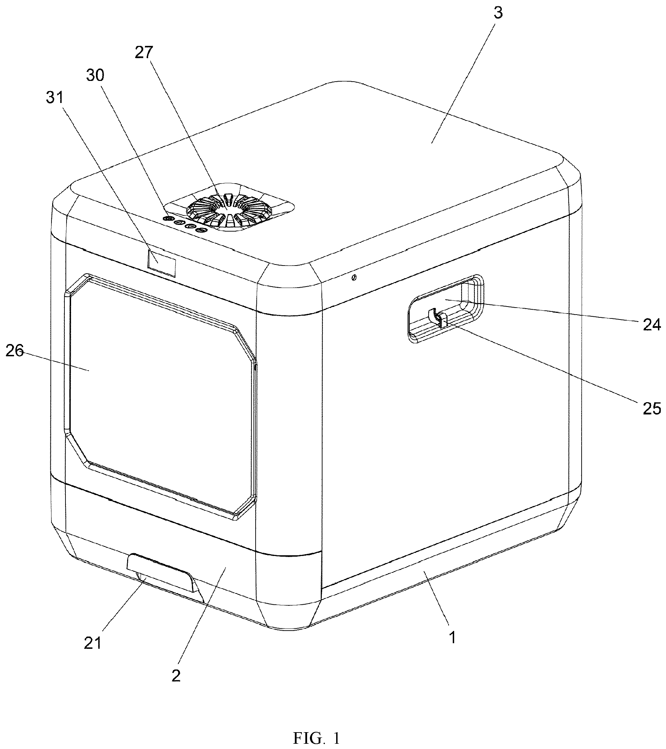

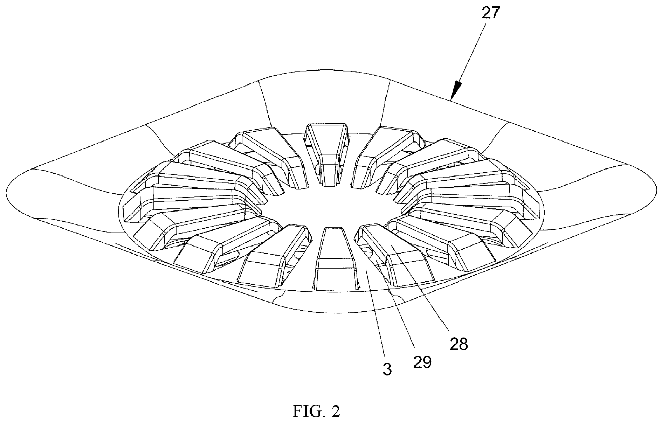

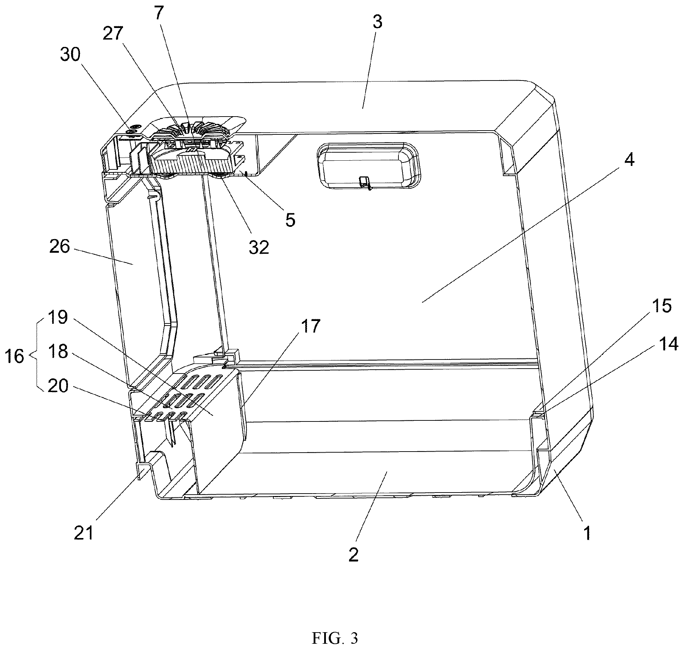

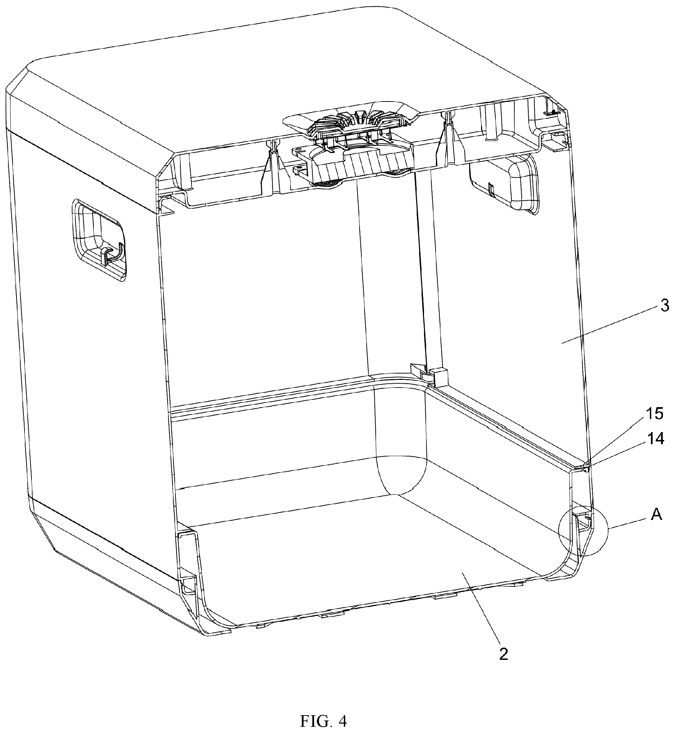

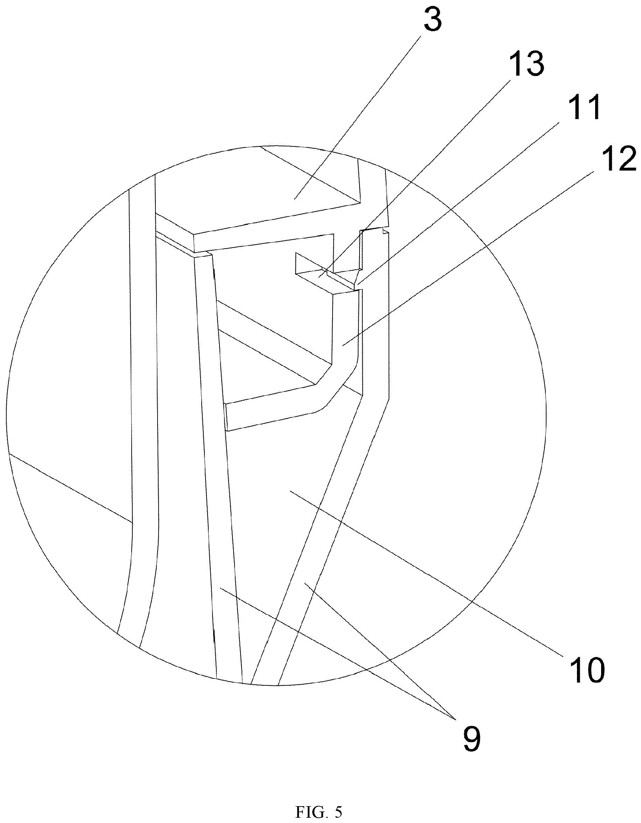

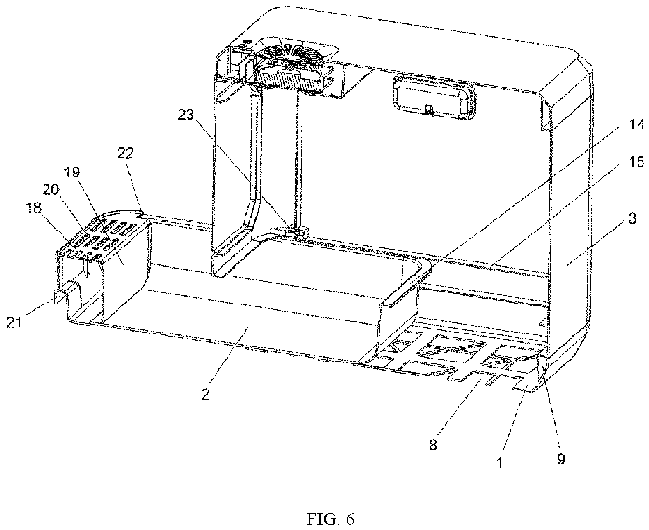

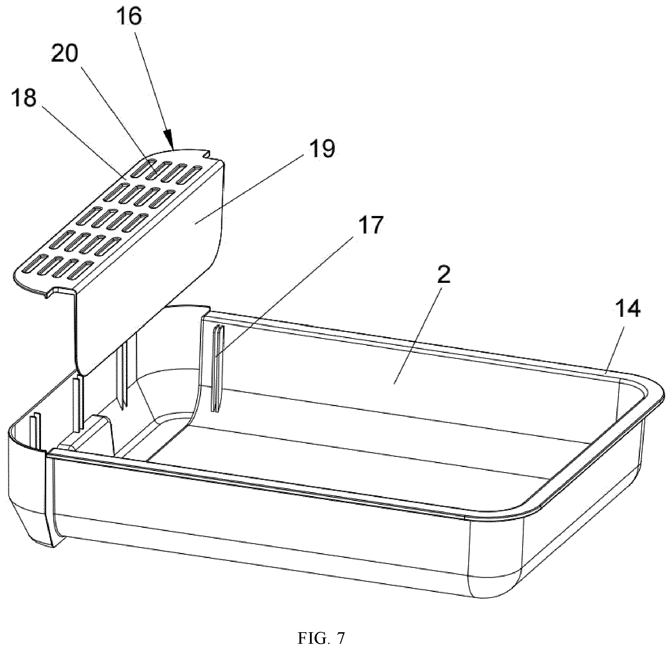

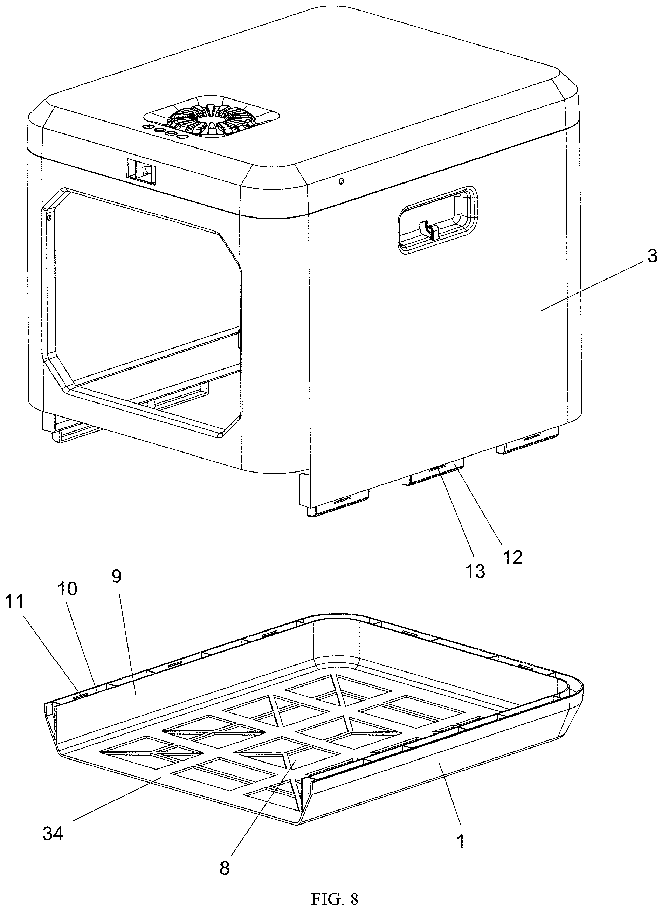

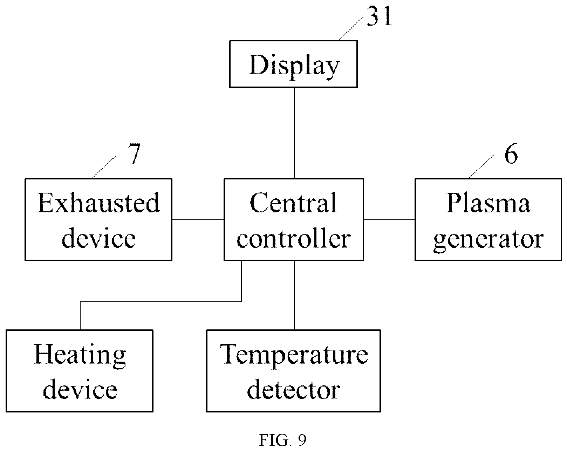

As shown in FIGS. 1 to 9, the present disclosure provide a plasma deodorant pet toilet, which comprises a base 1, a cat litter tray 2, an upper cover 3 and a deodorant and sterilization component, the upper cover 3 is installed above the base 1 and forms a basin space 4 with the base 1, the cat litter tray 2 is carried on the base 1, the base 1 is arranged with an open end 34, the cat litter tray 2 passes through the open end 34, and the deodorant and sterilization component comprises a plasma generator 6 and an exhaust device 7, the plasma generator 6 and the exhaust device 7 are both arranged on the upper cover 3, the plasma generator 6 is used to deodorize and sterilize the basin space 4, and the exhaust device 7 is used to blow the positive and negative ions generated by the plasma generator 6 to the basin space 4, makes the positive and negative ions evenly distributed in the basin space 4, which has a good deodorizing effect. Preferably, the exhaust device 7 is a fan; the upper cover 3 is a semi-wrapped upper cover.

The working principle of the plasma generator 6 is to raise the low voltage to positive high voltage and negative high voltage by the booster circuit, and use the positive high voltage and negative high voltage to ionize the air to generate a large amount of positive ion oxygen and negative ion oxygen. The higher the positive and negative voltage output, the greater the number of positive and negative ions produced. The generated positive ions and negative ions purify the air in the basin space 4 at the same time. After the positive and negative ions are neutralized with the air, a huge energy release is instantly generated, and the molecular structure changes rapidly, resulting in changes in bacterial structure or energy conversion, resulting in bacteria die and achieve their bactericidal effect. The number of negative ions in the present disclosure is far greater than the number of positive ions, and the remaining negative ions still float in the air during purification, which can eliminate smoke, remove dust, eliminate odor, improve air quality, promote blood circulation, and prevent bacteria in the basin space 4. The role of regeneration is beneficial to protect the health of humans and cats.

In practical applications, the present disclosure is also provided with a central controller, a display 31, and a temperature detector. The display 31, the temperature detector, the exhaust device 7 and the plasma generator 6 are all electrically connected to the central controller, and the display 31 is arranged on the upper cover 3 is easy to observe the temperature value, and the upper cover 3 is arranged with a plurality of buttons 30, and the buttons 30 are used to control the switch, the activation and closing of the plasma generator 6 and the exhaust device 7. Opening the exhaust device 7 can make the basin space 4 circulate air, and the ventilation effect is good, effectively preventing the growth of bacteria and reducing odor, and the deodorization effect is good. The cat litter tray 2 is a pull-out tray structure, which is convenient to replace cat litter, improves convenience, and has a good use experience. The upper cover 3 can be detachably connected by a plurality of plate-like structures, which is convenient for storage or transportation. The basin space 4 can be arranged with a heating device, which is preferably a heating plate, which can heat the basin space 4. In a relatively cold environment, the heating device can be selectively turned on to improve comfort and use experience.

In this embodiment, a side wall of the base 1 is double wall structure 9, the double wall structure 9 is arranged with inner groove 10, the inner groove 10 is arranged with a plurality of convex buckles 11, a lower end of the upper cover 3 is arranged with a plurality of pins 12, the pins 12 are inserted into the inner groove 10, and the convex buckles 11 are in one-to-one correspondence with the pins 12, the pins 12 are arranged with button holes 13, and the button holes 13 are fastened and installed with the convex buckles 11, which is not only firm, moreover, it is convenient to disassemble and assemble, which is beneficial to storage and transportation. The double-walled structure 9 enables the base 1 and the upper cover 3 to be installed firmly, and the appearance is well connected, which is more ornamental.

In this embodiment, an upper edge of the cat litter tray 2 is bent and arranged with folded edge 14, and an inner wall of the upper cover 3 is arranged with limit rib 15, the folded edge 14 is located under the limit rib 15, and the limit rib 15 is used to limit the position of the cat litter tray 2. It is the cat's instinctive reaction to plan sand after defecation. The limit rib 15 can effectively fix the cat litter tray 2, improve the stability, and provide a good user experience.

In this embodiment, the cat litter tray 2 is arranged with a filter sand part 16, the cat litter tray 2 is arranged with two installation slots 17, one installation slot 17 is arranged on one inner side wall of the cat litter tray 2, and the other installation slot 17 is arranged on the other inner side wall of the cat litter tray 2, the filter sand part 16 is arranged with a filter plate 18 and a card plate 19, the card plate 19 is bent and arranged on the filter plate 18, the filter plate 18 is arranged with a plurality of filter holes 20, one end of the card plate 19 protrudes into one installation slot 17, and the other end of the card plate 19 protrudes into the other installation slot 17. After the cat defecates, a small amount of cat litter will be entrained on the bottom of its feet. When the cat leaves the cat litter tray 2, the cat's claws step on the filter plate 18, which can filter the cat litter on the cat's claws and prevent the cat from taking the cat litter out of the outside, it is not easy to clean; the design of the filter plate 18 can recycle the cat litter. When the cat litter collected under the filter plate 18 reaches a certain amount, the entire filter sand part 16 and the cat litter tray 2 can be separated, and the filter plate 18 can be separated. The collected cat litter is transferred back to the cat litter tray 2, which can prevent the cat litter from being wasted and reduce the feeding cost.

In this embodiment, an outside of the cat litter tray 2 is arranged with a handle groove 21, and the handle groove 21 is a hidden groove structure with an opening downward. The hidden groove structure is not only beautiful, but also avoids the accumulation of cat hair, dust, debris, and so on compared to traditional handles.

In this embodiment, the cat litter tray 2 is arranged with a stop flange 22, the upper cover 3 is arranged with a stop sidewall 23, and the stop flange 22 is abutted against the stop sidewall 23. The design of the stop flange 22 and the stop sidewall 23 is used to control the depth of the cat litter tray 2 protruding into the basin space 4, which can make the cat litter tray 2 have a good closing effect, smooth appearance lines, and more ornamental.

In this embodiment, both side walls of the upper cover 3 are concavely arranged with a cavity 24, and the cavity 24 is arranged with a hook 25, and the hook 25 is used for hanging a cat litter shovel. When carrying the cat litter box, both hands can protrude into the cavity 24 to carry and move in a balanced manner. The cat litter shovel is arranged on the side wall of the upper cover 3, and the structure design is ingenious, which improves the convenience and provides a good user experience.

In this embodiment, the upper cover 3 is rotatably installed with a door 26, the upper cover 3 is arranged with an inlet and outlet, and the door 26 is arranged at the inlet and outlet. The door 26 is convenient for the cat to enter and exit, and can make the basin space 4 form a relatively tight seal that is beneficial to the efficient and comprehensive sterilization of the plasma generator 6, and the sterilization effect is good.

In this embodiment, the upper cover 3 is arranged with a hidden air inlet 27, the upper cover 3 is arranged with a mounting plate 5, and the mounting plate 5 is arranged with an air outlet 32, the exhaust device 7 is arranged on the mounting plate 5, an intake end of the exhaust device 7 is arranged corresponding to the hidden air inlet 27, and an outlet end of the exhaust device 7 is arranged corresponding to the hidden air inlet 27, the air outlet is communicated with the basin space 4, the hidden air inlet 27 is formed by a circular array of a plurality of arched convex strips 28, and there is a ventilation vertical hole 29 between the arched convex strips 28 and a top wall of the upper cover 3, a plane where the vertical ventilation holes 29 are located cross or is vertically arranged with the top plane of the upper cover 3. Compared with the traditional flat through holes, the vertical ventilation holes 29 can well prevent the accumulation of dust, cat hair and sundries, and can play a good role in protecting the electronic components on the mounting board 5 and the exhaust device 7, prolong the service life, and the structure design of the hidden air inlet 27 is ingenious, the design is full of aesthetics, and it is easier to be favored by cat owners.

In this embodiment, a bottom wall of the base 1 is arranged with a plurality of through holes 8, which not only enables the basin space 4 to be ventilated and ventilated, but also saves manufacturing materials and reduces production costs.

All the technical features in this embodiment can be freely combined according to actual needs.

The above-mentioned embodiment is a preferred implementation scheme of the present disclosure. In addition, the present disclosure can also be implemented in other ways, and any obvious replacements are within the protection scope of the present disclosure without departing from the concept of the technical solution.

## Claims

1. A plasma deodorant pet toilet, comprising a base (1), **characterized by** further comprising:
a cat litter tray (2), the cat litter tray (2) is carried on the base (1), the base (1) is arranged with an open end, the cat litter tray (2) passes through the open end;
an upper cover (3), the upper cover (3) is installed above the base (1) and forms a basin space (4) with the base (1);
and a deodorant and sterilization component, the deodorant and sterilization component comprise a plasma generator (6) and an exhaust device (7), the plasma generator (6) and the exhaust device (7) are both arranged on the upper cover, the plasma generator (6) is used to deodorize and sterilize the basin space (4), and the exhaust device (7) is used to blow the positive and negative ions generated by the plasma generator (6) to the basin space (4);
an outside of the cat litter tray (2) is arranged with a handle groove (21), and the handle groove (21) is a hidden groove structure with an opening downward;
the cat litter tray (2) is arranged with a stop flange (22), the upper cover (3) is arranged with a stop sidewall (23), and the stop flange (22) is abutted against the stop sidewall (23).

2. The plasma deodorant pet toilet according to claim 1, wherein a side wall of the base (1) is double wall structure (9), the double wall structure (9) is arranged with inner groove (10), the inner groove (10) is arranged with a plurality of convex buckles (11), a lower end of the upper cover (3) is arranged with a plurality of pins (12), the pins (12) are inserted into the inner groove (10), and the convex buckles (11) are in one-to-one correspondence with the pins (12), the pins (12) are arranged with button holes (13), and the button holes (13) are fastened and installed with the convex buckles (11).

3. The plasma deodorant pet toilet according to claim 1, wherein an upper edge of the cat litter tray (2) is bent and arranged with folded edge (14), and an inner wall of the upper cover (3) is arranged with limit rib (15), the folded edge (14) is located under the limit rib (15), and the limit rib (15) is used to limit the position of the cat litter tray (2).

4. The plasma deodorant pet toilet according to claim 1, wherein the cat litter tray (2) is arranged with a filter sand part (16), the cat litter tray (2) is arranged with two installation slots (17), one installation slot (17) is arranged on one inner side wall of the cat litter tray (2), and the other installation slot (17) is arranged on the other inner side wall of the cat litter tray (2), the filter sand part (16) is arranged with a filter plate (18) and a card plate (19), the card plate (19) is bent and arranged on the filter plate (18), the filter plate (18) is arranged with a plurality of filter holes (20), one end of the card plate (19) protrudes into one installation slot (17), and the other end of the card plate (19) protrudes into the other installation slot (17).

5. The plasma deodorant pet toilet according to claim 1, wherein both side walls of the upper cover (3) are concavely arranged with a cavity (24), and the cavity (24) is arranged with a hook (25), and the hook (25) is used for hanging a cat litter shovel.

6. The plasma deodorant pet toilet according to claim 1, wherein the upper cover (3) is rotatably installed with a door (26), the upper cover (3) is arranged with an inlet and outlet, and the door (26) is arranged at the inlet and outlet.

7. The plasma deodorant pet toilet according to claim 1, wherein the upper cover (3) is arranged with a hidden air inlet (27), the upper cover (3) is arranged with a mounting plate (5), and the mounting plate (5) is arranged with an air outlet (32), the exhaust device (7) is arranged on the mounting plate (5), an intake end of the exhaust device (7) is arranged corresponding to the hidden air inlet (27), and an outlet end of the exhaust device (7) is arranged corresponding to the hidden air inlet (27), the air outlet is communicated with the basin space (4), the hidden air inlet (27) is formed by a circular array of a plurality of arched convex strips (28), and there is a ventilation vertical hole (29) between the arched convex strips (28) and a top wall of the upper cover (3), a plane where the vertical ventilation holes (29) are located is arranged intersecting with the top plane of the upper cover (3).

8. The plasma deodorant pet toilet according to claim 1, wherein a bottom wall of the base (1) is arranged with a plurality of through holes (8).

## Amended claims

### Amended claims in accordance with Rule 137(2) EPC.

1. A plasma deodorant pet toilet, comprising:
a base (1),
a cat litter tray (2), the cat litter tray (2) is carried on the base (1), the base (1) is arranged with an open end, the cat litter tray (2) passes through the open end and is arranged with a stop flange (22);
an upper cover (3), the upper cover (3) is installed above the base (1) and forms a basin space (4) with the base (1);
and a deodorant and sterilization component,
the deodorant and sterilization component comprise a plasma generator (6) and an exhaust device (7), the plasma generator (6) and the exhaust device (7) are both arranged on the upper cover, and the exhaust device (7) is used to blow the positive and negative ions generated by the plasma generator (6) to the basin space (4);
**characterized in that**
the plasma generator (6) is configured to generate positive ions of oxygen and negative ions of oxygen such that the number of the negative ions is greater than the number of the positive ions, so as to deodorize and sterilize the basin space (4);
an outside of the cat litter tray (2) is arranged with a handle groove (21), and the handle groove (21) is a hidden groove structure with an opening downward;
the upper cover (3) is arranged with a stop sidewall (23), and the stop flange (22) is abutted against the stop sidewall (23).

2. The plasma deodorant pet toilet according to claim 1, wherein a side wall of the base (1) is double wall structure (9), the double wall structure (9) is arranged with inner groove (10), the inner groove (10) is arranged with a plurality of convex buckles (11), a lower end of the upper cover (3) is arranged with a plurality of pins (12), the pins (12) are inserted into the inner groove (10), and the convex buckles (11) are in one-to-one correspondence with the pins (12), the pins (12) are arranged with button holes (13), and the button holes (13) are fastened and installed with the convex buckles (11).

3. The plasma deodorant pet toilet according to claim 1, wherein an upper edge of the cat litter tray (2) is bent and arranged with folded edge (14), and an inner wall of the upper cover (3) is arranged with limit rib (15), the folded edge (14) is located under the limit rib (15), and the limit rib (15) is used to limit the position of the cat litter tray (2).

4. The plasma deodorant pet toilet according to claim 1, wherein the cat litter tray (2) is arranged with a filter sand part (16), the cat litter tray (2) is arranged with two installation slots (17), one installation slot (17) is arranged on one inner side wall of the cat litter tray (2), and the other installation slot (17) is arranged on the other inner side wall of the cat litter tray (2), the filter sand part (16) is arranged with a filter plate (18) and a card plate (19), the card plate (19) is bent and arranged on the filter plate (18), the filter plate (18) is arranged with a plurality of filter holes (20), one end of the card plate (19) protrudes into one installation slot (17), and the other end of the card plate (19) protrudes into the other installation slot (17).

5. The plasma deodorant pet toilet according to claim 1, wherein both side walls of the upper cover (3) are concavely arranged with a cavity (24), and the cavity (24) is arranged with a hook (25), and the hook (25) is used for hanging a cat litter shovel.

6. The plasma deodorant pet toilet according to claim 1, wherein the upper cover (3) is rotatably installed with a door (26), the upper cover (3) is arranged with an inlet and outlet, and the door (26) is arranged at the inlet and outlet.

7. The plasma deodorant pet toilet according to claim 1, wherein the upper cover (3) is arranged with a hidden air inlet (27), the upper cover (3) is arranged with a mounting plate (5), and the mounting plate (5) is arranged with an air outlet (32), the exhaust device (7) is arranged on the mounting plate (5), an intake end of the exhaust device (7) is arranged corresponding to the hidden air inlet (27), and an outlet end of the exhaust device (7) is arranged corresponding to the hidden air inlet (27), the air outlet is communicated with the basin space (4), the hidden air inlet (27) is formed by a circular array of a plurality of arched convex strips (28), and there is a ventilation vertical hole (29) between the arched convex strips (28) and a top wall of the upper cover (3), a plane where the vertical ventilation holes (29) are located is arranged intersecting with the top plane of the upper cover (3).

8. The plasma deodorant pet toilet according to claim 1, wherein a bottom wall of the base (1) is arranged with a plurality of through holes (8).
